# EUROPEAN PATENT SPECIFICATION

(11) **EP 0 669 000 B1**
(45) Date of publication and mention of the grant of the patent: **07.05.1997**
(21) Application number: 94900067.3
(22) Date of filing: 15.11.1993
(51) Int. Cl.: G01N 33/543, G01N 33/68

(54) **TWO-SITE IMMUNOASSAY FOR AN ANTIBODY WITH CHEMILUMINESCENT LABEL AND BIOTIN BOUND LIGAND**
ZWEISEITEN-IMMUNOASSAY FÜR EINEN ANTIKÖRPER MIT CHEMILUMINESZIERENDEM MARKER UND BIOTIN-GEBUNDENEM LIGANDEN
TITRAGE IMMUNOLOGIQUE D'UN ANTICORPS SUR DEUX SITES, AVEC MARQUAGE CHIMIOLUMINESCENT ET LIGAND LIE A LA BIOTINE

(30) Priority: 13.11.1992 DK 1379/92
(43) Date of publication of application: 30.08.1995
(73) Proprietor: ALK A/S, 2970 Hoersholm (DK)
(72) Inventor: JOHANSEN, Niels, DK-3450 Aller d (DK); IPSEN, Hans-Henrik, DK-3400 Hiller d (DK)
(74) Representative: Kyed, Iver
(86) International application number: DK9300373
(87) International publication number: WO9411734

(56) References cited:
- CLINICAL & EXPERIMENTAL ALLERGY, vol. 22, 1992; J. KLEINE-TEBBE et al., pp. 475-484
- METHODS IN ENZYMOLOGY, vol. 73, 1981; J.-L. GUESDON et al., p. 471
- JOURNAL OF IMMUNOLOGICAL METHODS, vol. 116, 1989; M. SAKAGUCHI et al., pp. 181-187
- NATL. LIBRARY OF MEDICINE, File Medline; R.C. HART, AN 87281685
- NATL. LIBRARY OF MEDICINE, File Medline; F.J. BAGAZGOITIA, AN 89102665
- METHODS IN ENZYMOLOGY, vol. 184, 1990; C.J. STRASBURGER et al., pp. 481-496

## Description

The present invention relates to a method of detecting an antibody in a sample using a chemiluminescent labelling compound.

More specifically, the invention relates to the use of a chemiluminescent acridinium ester compound coupled to avidin or streptavidin, and a ligand coupled to biotin in a two-site immunoassay wherein the affinity complex is captured on paramagnetic particles, which makes possible a rapid detection and/or quantification of immunologically active substances, such as antibodies in samples such as biological fluids and tissue samples, milk, food samples, beverages, water or industrial effluents.

A method of detecting and quantifying immunoglobulin E-antibodies in serum is disclosed in the brochure "Specific IgE, Magic® Lite SQ™, published by Ciba Corning Diagnostics Corp. and ALK Laboratories in September 1990. In said method a specific allergen covalently bound to paramagnetic particles reacts with the allergen-specific IgE-antibody in a serum or plasma sample. After a first incubation period and washing away unbound non-specific IgE, a chemiluminescent acridinium ester-labelled monoclonal antibody against IgE is added. Following a second incubation period the solid phase bound and labelled antibody is measured in a Magic Lite® Analyzer (Cat. No. 472733 or Cat. No. 472270) which automatically injects reagents, which initiate the chemiluminescent reaction. When using said method only the final step of initiating and measuring the chemiluminescence can be automated. The chemiluminescent acridinium ester labelling compound is described in US patent No. 4,745,181.

US patent No. 4,946,958 discloses a chemiluminescent acridinium ester linked to an N-succinimidyl moiety which can be further linked to a protein or polypeptide to provide an immunologically reactive luminescent reagent. Said reagent can be used in an immuno-assay, which may involve the simultaneous binding of a solid phase antibody, an antigen molecule and a labelled anti-antibody, separation and washing of the solid phase and quantifying the luminescence of the solid phase.

Strasburger et al. discloses in Methods in Enzymology, 184(1990), pp 481-496 the use of a two-site chemiluminescent immuno-assay, wherein hGH and hCG hormones are captured by antibodies immobilized on microtiter plates and labelled with a chemiluminescent agent coupled to avidin through a biotin labelled second antibody. Antigenic analytes, such as protein hormones, may be assayed directly from serum samples and compared to standard curves. The concentration of immunoglobulins, such as IgE, is, however, extremely patient dependent and an assay of a specific IgE must be compared with the individual patient level of total IgE and therefore reuqires an assay having a greater dynamic range than is obtainable in the assay disclosed by Strasburger et al.

EP-A-0 425 217 discloses a hybridization assay wherein a chemiluminescent complex is formed comprising a nucleic acid hybridised with a first labelled nucleotide probe coupled to paramagnetic particles and a second nucleotide probe labelled with biotin and coupled to an avidin-acridinium ester. However, the person skilled in the art confronted with the problem of providing a fully automated method of detecting antibodies will search for a method which can be carried out in one reaction container and preferably under ambient reaction conditions. The assay represented here is fundamentally different as it employs detection of specific nucleotide sequences, which are not antigens towards which specific antibodies can be raised. More detailed it is necessary in said assay to use elevated temperatures for the hybridization and a hapten-oligonucleotide probe which is not necessary nor desirable in the immuno-assays.

Until now immuno-assays for the quantification of immunologically active molecules, such as immunoglobulins (e.g. specific immunoglobulin-E), in biological fluids, such as serum, have been manual, e.g. the Enzyme Linked Immuno Sorbent Assay (ELISA), or semi-automatical. And the typical duration of a semi-automatical immuno-assay, such as the Magic® Lite SQ™ specific IgE assay referred to above, is approximately two hours.

Moreover, commercial specific IgE assays (CAP,RAST, supplied by Pharmacia, Uppsala) and the Magic® Lite SQ™ specific IgE assay use a total-IgE reference assay having a non-identical protocol resulting in unprecise data. Only assays using the same catching and detection procedures are directly comparable. For example all specific IgE close response curves must be parallel with total IgE response curves. The reason is that the required dynamic range for a specific (or total?) IgE assay is 2 decades and the required dynamic range for a total IgE assay is from 2 to 7 decades, and the existing immuno-assays do not allow concentration measurements over the entire range. Thus, until now it has been necessary to use different protocols for the specific and total immunoglobulin assays having different reagents.

Because of the increasing interest in safe laboratory procedures there is a need for a fully automated method of detecting substances, such as antibodies, in biological fluids, such as human serum, plasma, blood, milk, urine or saliva, which method should provide a minimised risk of contact with hazardous fluids. Also, the increasing use of laboratory tests in diagnostics calls for methods of short duration, preferably only a few minutes.

It is therefore an object of this invention to provide a method of detecting an antibody in a sample, which method is safe, rapid, and fully automatable.

This object is achieved by one method according to the invention which method is characterised in:
a) mixing a ligand antigen, antibody, or hapten bound to biotin or a functional derivative thereof; an antibody directed against the antibody to be detected bound to paramagnetic particles; and a chemiluminescent acridinium compound bound to avidin, streptavidin or a functional derivative thereof with the sample to form a solid phase bound complex,
b) magnetically separating the solid phase from the liquid phase,
c) initiating the chemiluminescent reaction, and analysing the separated solid phase for the presence of chemiluminescent complex, wherein the presence of chemiluminescence is an indication of the presence of said antibody in said sample.

Although the method according to the invention can be carried out by the above defined steps (a), (b) and (c) it is preferred to add the labelling compound in a separate step, and the method is preferably carried out according to the following steps:
i) mixing the ligand antigen, antibody, or hapten bound to biotin or a functional derivative thereof with the sample and the antibody directed against the antibody to be detected bound to paramagnetic particles to form a first solid phase complex,
ii) adding a chemiluminescent acridinium compound covalently bound to avidin, streptavidin or a functional derivative thereof to form a second solid phase complex,
iii) magnetically separating the solid phase from the liquid phase;
iv) initiating the chemiluminescent reaction, and analysing the separated solid phase for the presence of chemiluminescent complex.

A particular object of the invention is to provide a fully automatable immuno-assay for the quantification of specific antibodies, such as immunoglobulins, wherein a truly parallel reference immuno-assay using an identical protocol is used as the reference.

The object of quantifying specific antibodies using a truly parallel reference immuno-assay is achieved by a method of measuring the concentration and/or the relative contents of a specific antibody in a liquid sample, wherein the measured light emission of a separated solid phase comprising a captured specific antibody coupled to a chemiluminescent label is compared with the measured light emission obtained in a parallel reference immuno-assay wherein the total contents of the class of antibodies in the sample to which said specific antibody belongs is measured, said method comprising the steps of
a) mixing a ligand antigen or hapten towards which the specific antibody to be measured is directed bound to biotin or a functional derivative thereof; an antibody directed against the constant portion of the antibody to be measured bound to paramagnetic particles; and a chemiluminescent acridinium compound bound to avidin, streptavidin or a functional derivative thereof with the sample to form a first solid phase complex,
b) magnetically separating said first solid phase from the liquid phase,
c) initiating a chemiluminescent reaction and measuring the light emission of the separated first solid phase,
d) mixing a ligand antibody directed against the class of antibodies to be measured bound to biotin or a functional derivative thereof; an antibody directed against the constant portion of the class of antibodies to be measured bound to paramagnetic particles; and a chemiluminescent acridinium compound bound to avidin, streptavidin or a functional derivative thereof with the sample to form a second solid phase complex,
e) magnetically separating said second solid phase from the liquid phase,
f) initiating the chemiluminescence reaction and measuring the light emission of the separated second solid phase, and
g) comparing the light emission of the separated first solid phase with that of the separated second solid phase.

In a preferred embodiment of the method described above step a) is performed by
(i) mixing the ligand antigen or hepten bound to bioten or a functional derivative thereof with the sample and the antibody bound to paramagnetic particles to form a solid phase complex, and
(ii) adding the chemiluminescent acridinium compound bound to avidin, streptavidin or a functional derivative thereof to form said first solid phase complex,
and step d) is performed by
(i) mixing the ligand antibody bound to biotin or a functional derivative thereof with the sample and the antibody bound to paramagnetic particles to form a solid phase complex and
(ii) adding the chemiluminescent acridinium compound covalently bound to avidin, streptavidin or a functional derivative thereof to form said second solid phase complex.

The specific antibody to be measured in the sample is preferably a specific immunoglobulin selected from the group consisting of IgA, IgD, IgE, IgG, IgM, and isotypes thereof, and the ligand antigen, antibody, or hapten directed against the variable portion of said antibody is an allergen, and the class of antibodies is preferably a class of immunoglobulins selected from the group consisting of total IgA, total IgD, total IgE, total IgG, total IgM, and isotypes thereof, and the ligand antigen, antibody or hapten is an antibody directed against said class of immunoglobulins.

More preferably the specific immunoglobulin is a specific IgE, and the class of antibodies is total IgE.

The antibody directed against the antibody to be measured bound to paramagnetic particles is preferably selected from the group consisting of polyclonal antibodies, monoclonal antibodies including recombinant antibodies, fragmented antibodies, preferably a monoclonal mouse anti-immunoglobulin.

The advantages of the invention are:
- All reagents can be mixed simultaneously in one reaction container, which minimises the risk of contamination, errors and operation steps, reduces significantly the duration of the immuno-assay, and greatly facilitates automation of the process and precision is improved, cf. example 6;
- Quantification of specific antibodies in, e.g. a serum sample, can be performed with reference to a truly parallel total antibody assay using an identical protocol, cf. example 3;
- The obtained greater capacity and sensitivity facilitates that even very low concentrations of immunoglobulins and low concentrations of specific immunoglobulins can be detected, cf. examples 1, 2 and 7.

A preferred embodiment of the invention is an immuno-assay for the detection of antibodies, such as specific immunoglobulins (IgA, IgE, IgG, IgM and isotypes thereof) in a sample, such as serum or saliva.

Particularly, the invention can be used in an assay for the detection and quantification of specific IgE in a sample. When the sample is liquid, e.g. serum or plasma, it can be added directly to a reaction container comprising preferably a monoclonal mouse anti-IgE antibody bound to suspended paramagnetic particles and a specific allergen (ligand) bound to biotin in an aqueous medium. The biotin is preferably biotin amidocaproate N-hydroxysuccinimide ester (Sigma Catalog No. B2643) When the sample is non-liquid, e.g. a tissue sample, it is preferably homogenised and suspended in an aqueous liquid. A simultaneous reaction between specific IgE in the sample, allergen and monoclonal anti-IgE antibody in the aqueous medium results in the formation of a conjugate. A chemiluminescent labelling compound, preferably an acridinium ester coupled to streptavidin (Sigma Catalog No. S4762) (avidin-DMAE) is added to the reaction container and a binding reaction between avidin-DMAE and biotin bound to the conjugate and unconjugated allergen-bound biotin takes place. The conjugate bound label is separated from the unbound DMAE-labelled antibody by magnetically separating the reaction mixture and decanting the supernatant. The chemiluminescence of the separated conjugate is measured as described in Pazzagli, M. et al. (eds.). Studies and applications in "Biology & Medicine", Journal of Bioluminescence and Chemiluminescence 4(1), 1-646, 1989.

As a reference, a truly parallel total-IgE immuno-assay differing only in that a preferably polyclonal anti-IgE antibody bound to biotin is used as the ligand is performed simultaneously.

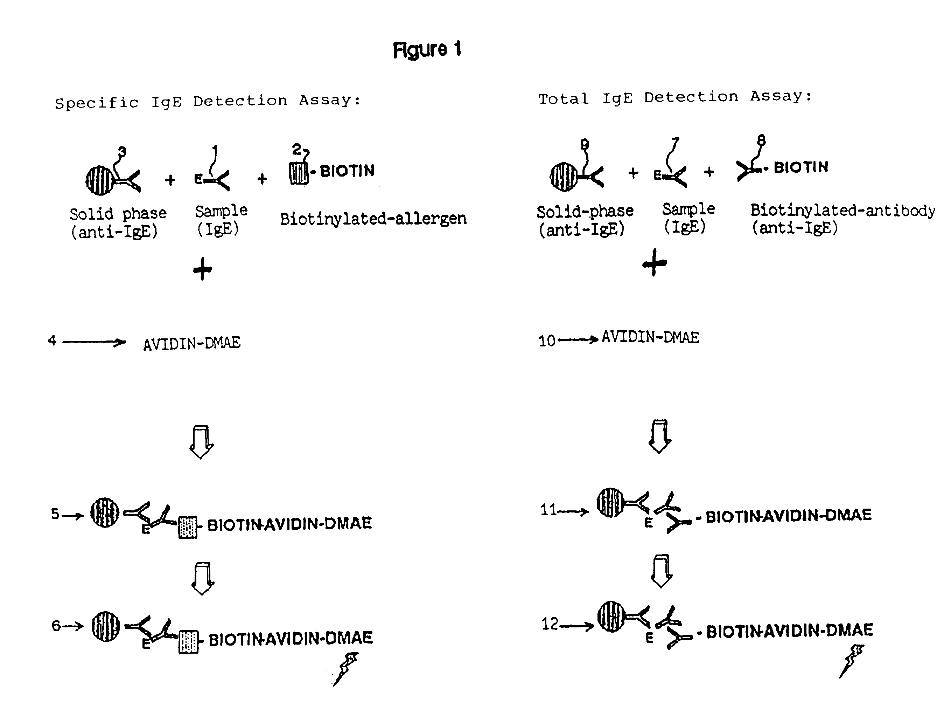

Figure 1 is a diagrammatic representation of a specific IgE assay according to the invention and comprising a parallel total IgE reference assay. In the figure (1) represents the specific IgE-antibody to be detected, (2) is a specific allergen bound to biotin, (3) is a monoclonal mouse anti-IgE bound to paramagnetic particles, (4) is an avidin-acridinium ester and (5) represents the solid phase labelled complex formed between (1), (2), (3) and (4) and includes optional incubation, separation and optional washing steps, and (6) represents a final step of initiating the chemiluminescent reaction and measuring the light emission.

In the total IgE reference assay in Figure 1 (7) represents IgE (WHO 75/502 IU/ml), (8) is polyclonal anti-IgE bound to biotin, (9) is monoclonal mouse anti-IgE bound to suspended paramagnetic particles, (10) is an avidin-acridinium ester and (11) represents the solid phase labelled complex formed between (7), (8), (9) and (10) and includes optional incubation, separation and optional washing steps, and (12) is the final step of initiating the chemiluminescent reaction and measuring the light emission.

More particularly, the immuno-assay using the method of the invention can be performed in ACS:180 fully automatic analyzer produced by Ciba Corning Diagnostics Corp., Medfield, Mass., U.S.A.

In one preferred embodiment of the invention the immunologically active substance to be detected is an antibody, e.g. against penicillin or derivatives thereof, such as benzylpenicillin, penicilloyl, etc., and the ligand bound to biotin is a hapten, such as penicillin or derivatives thereof.

### Definitions

In the methods of the invention the antibody to be detected is a specific immunoglobulin, preferably a specific IgA, IgD, IgE, IgG, IgM, and isotypes thereof, and more preferably a specific IgE, or a class of antibodies, such as immunoglobulins, preferably selected from the group consisting of total IgA, total IgD, total IgE, total IgG, total IgM and isotypes thereof, most preferably total IgE.

By sample is meant any liquid or liquefied sample, including solutions, emulsions, dispersions and suspensions.

The ligand antigen, antibody or hapten bound to biotin can be any immunologically active substance, such as an allergen, antibodies, such as polyclonal antibodies, monoclonal antibodies including recombinant antibodies or fragmented antibodies, preferably an allergen and/or a polyclonal anti-immunoglobulin, such as goat anti-human polyclonal serum supplied by Ventrex Laboratories, Inc., Portland, Maine, Catalog No. 77660. In the reference immuno-assay said antibody is preferably directed against the constant portion of the class of antibodies to be measured, i.e. an antibody directed against the IgE-antibodies.

By biological fluid is meant any clinical sample, such as blood, plasma, serum, urine or saliva, which also includes any biological fluid which is excreted, secreted or transported internally in an organism.

By paramagnetic particles (PMP) is meant particles which can be dispersed or suspended in a liquid medium. Throughout the examples are used BioMag™ particles (iron oxide particles coated with amine terminated groups) sold by advanced Magnetics Inc., Cambridge, Massachusetts. The antibodies coupled to PMP are preferably directed against the constant portion of the antibodies to be detected or measured and may be polyclonal or monoclonal antibodies including recombinant or fragmented antibodies, preferably a monoclonal antibody, MAb A 5697-1A3(920325) supplied by BioInvent International AB, Lund, Sweden.

The chemiluminescent acridinium compound is preferably N-hydroxysuccinimide dimethylacridinium ester covalently bound to avidin or streptavidin (avidin-DMAE). Avidin and DMAE are coupled according to the methods of Weeks et al., Clin. Chem. 29/8, 1474-1479 (1983). Other luminescent labelling compounds that can be bound to avidin or streptavidin may be used in the method of the invention. E.g. luminol, lucigenin or lophine.

### Preparation of biotinylated antibodies

### Biotinylated anti-IgE and Phleum pratense:

Goat anti-human polyclonal serum (Ventrex Laboratories, Inc. MA, USA) is purified by affinity chromatography on a CNBr-activated sepharose™ 4B (Pharmacia, Uppsala, Sweden) with myeloma IgE (OEM concepts, USA) as a ligand. The anti-IgE is biotinylated with the ratio mol biotin: mol anti-IgE = 41:1.

9 µl of Biotin (Biotin amidocaproate N-hydroxysuccinimide ester (Sigma) 25 mg/ml in Dimethylformamide (Merck) is added to 0.4 ml of anti-IgE 4.5 mg/ml in 0.1 M NaHCO₃ (Merck). The reagents are incubated in an "end over end" mixer for 2 hours at 25°C. 0.9 ml lysin (Sigma) solution 20 mg/ml NaHCO₃ is added. The solution is filtered and the biotinylated antibody is purified by size chromatography on superdex™ 75 Hiload 16/60 (Pharmacia, Uppsala, Sweden). The pooled fractions are diluted in phosphate buffered saline PBS, pH 7.2, containing 0.1 % human serum albumin (Sigma) 0.1 % NaN₃ (Sigma).

The Phleum pratense extract, (ALK Laboratories A/S, Hørsholm, Denmark) is biotinylated in the molar ratio of 10:1 0.65 ml of biotin 10 mg/ml is added to 0.43 ml of 10 mg/ml Phleum pratense in 0.1 M NaHCO₃. The reagents are incubated for 2 hours at 25°C in an "end over end" mixer, after the incubation 40 µl lysin (Sigma) solution 50 mg/ml is added. The solution is filtered and the biotinylated antibody is purified from excess of biotin by size exclusion chromatography on superdex™ 75 Hiload 16/60 (Pharmacia). The fractions containing the allergens are pooled. The biotinylated Phleum pratense is diluted with PBS pH 7.2, containing 0.1 % human serum albumin (Sigma) and 0.1 % NaN₃ (Sigma).

### Preparation of streptavidin-acridinium ester label

Streptavidin was conjugated with DMAE-NHS, [2',6'-dimethyl-4'-(N-succinimidyloxycarbonyl)phenyl-10-methylacridinium-9-carboxylate methosulphate] using the methods of Weeks et al., Clin.Chem. 29/8, 1474-1479 (1983).

### Preparation of Streptavidin-acridinium ester label:

0.96 mg N-hydroxysuccinimide dimethylacridinium ester DMAE (Ciba Corning Diagnostics Corp., Medfield, MA, USA) is diluted in 1.92 ml Dimethylformamide. 250 µl of this solution is pipetted to 2.5 ml 1 mg/ml streptavidin (Sigma) in 0.1 M sodium dihydrogenphosphate, 0.15 M NaCl pH 8.15.

The air above the solution in the vial is exchanged with nitrogen, (AGA). The reagents are incubated for 30 min at 25°C under stirring, after incubation 2250 µl 10 mg/ml lysin 0.1 M sodium dihydrogenphosphate (Merck), 0.15 M NaCl (Merck) pH 8.15 is added. To remove unbound DMAE the solution is loaded on a PD-10™ column (Pharmacia, Uppsala, Sweden). The eluate is collected and purified by ultrafiltration using a cellulose Minitan-S™ filter sheet 10.000 NMWL (Millipore). The filtration is performed with 1.5 l phosphate buffered saline, PBS pH 7.2. The retentate (retanate) is concentrated to 25 ml and 25 ml PBS pH 7.2 containing 0.5 % HSA (Sigma) and 0.1 % NaN₃ (Sigma) is added.

The invention will be described in detail in the following examples.

### Immobilisation of antibody to paramagnetic particles

6.5 g paramagnetics particles (Ciba Corning Diagnostics Corp., MA, USA) are washed in 650 ml methanol (Merck) 3 times using magnetic separation. A wash with 650 ml 0.01 M acetate buffer pH 5.5 is performed twice. The particles are activated in 6.25 % glutaraldehyde (Merck), 0.01 M acetate buffer pH 5.5 for 3 hours at 25°C. The particles are washed 3 times in 650 ml 0.01 M acetate buffer pH 5.5. The particles are coupled with 1083 mg monoclonal anti-IgE antibody (ALK Laboratories, Hørsholm, Denmark) specific against the IgE Fc domain, for 24 hours at 25°C. The particles are washed twice in 0.01 M acetate buffer pH 5.5. Blocking of excess of active groups is performed with 200 ml 10 % IgE stripped serum (ALK Laboratories, Hørsholm, Denmark) for 24 hours at 25°C. The particles are washed in 650 ml 0.01 M phosphate buffer (Merck) followed by 3 washes in 650 ml 1 M NaCl (Merck). The particles are washed 3 times in 0.01 M phosphate buffer. The particles are resuspended in 650 ml PBS pH 7.2, 0.1 % w/v bovine serum albumine (Sigma), 0.001 % bovine gamma globulin (Sigma) and heat treated for 18 hours at 50°C. The particles are washed 3 times in 650 ml PBS pH 7.2, 0.1 % w/v bovine serum albumine (Sigma), 0.001 % bovine gamma globulin (Sigma). The particles are heat treated for 7 days at 37°C. The particles are washed in 0.01 M phosphate buffer twice. The particles are diluted to 0.5 g per 1 in PBS pH 7.2, 0.5 % human serum albumine (Sigma).

### Example 1

### Detection and quantification of antigen (total IgE)

Determination of total IgE antibodies, according to the invention, was conducted on Ciba Corning ACS:180 Benchtop Immunoassay analyzer described in Clinical Chemistry, 36/9, 1598-1602 (1990), using the following protocol:

50 µl of sample and 50 µl biotinylated anti-IgE are dispensed by the sample probe into the cuvette. The cuvette reaches the first reagent probe R1, where 100 µl paramagnetic particles with immobilised monoclonal anti-IgE antibody (ALK Laboratories A/S, Hørsholm, Denmark) specific against the IgE Fc domain are dispensed together with 200 µl of streptavidin-acridinium ester label (ALK Laboratories A/S, Hørsholm, Denmark). The cuvette moves down the track to the magnets and wash station. Wash with 750 µl deionized water is performed twice. After completion of the wash cycle the particles are resuspended in 300 µl 0.5 g/l H₂O₂ in 0.1 M HNO₃. The cuvette enters the luminometer chamber and in front of the photomultiplier 300 µl 25 mM NaOH solution is added and the photons of light emitted are measured and quantitated and expressed as relative light units (RLU). The amount of RLU is directly proportional to the amount of the IgE in the sample. The time from sample dispension to first result is 15 min and a new result follows every 20 second. Results were expressed as RLU experiment/RLU background, where RLU background was the chemiluminescent reaction observed in the absence of total IgE.

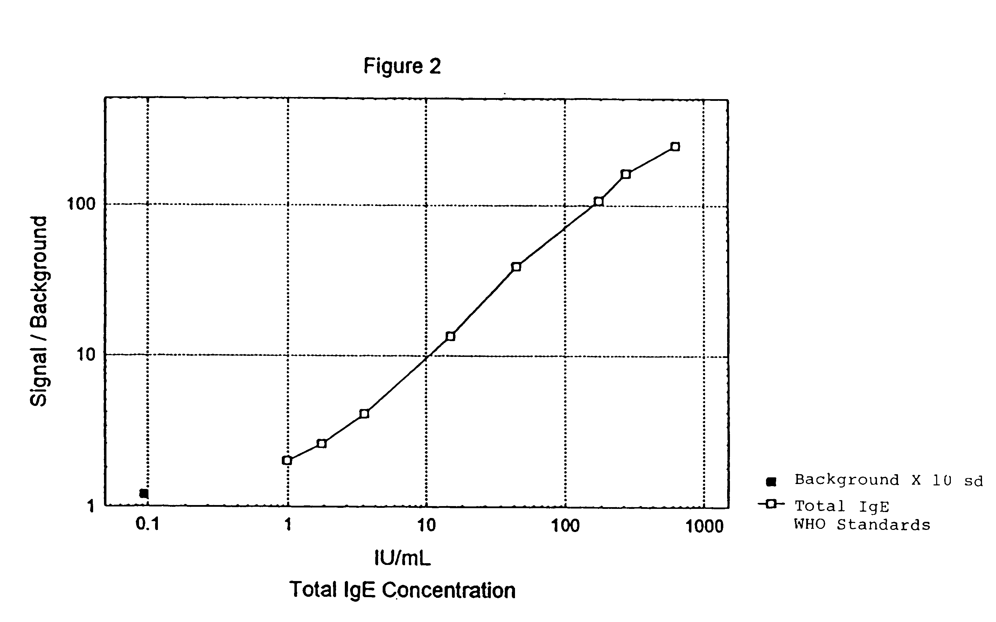

Nine Total IgE standards, calibrated in Magic Lite Total IgE Kit (ALK Laboratories A/S, Hørsholm, Denmark) against WHO 2nd IRP no 75/502 for human serum IgE, were assayed using the protocol described above and it is shown that 0.1 IU/ml of total serum IgE could be detected (as determined by background x 10 standard deviations), see Figure 2.

### Example 2

### Detection and quantification of specific antibody (Specific IgE)

Determination of Phleum pratense specific IgE antibodies (timothy grass specific IgE, according to the invention, was conducted on Ciba Corning ACS:180 Benchtop Immunoassay analyzer described in Clinical Chemistry, 36/9, 1598-1602 (1990), using the following protocol:

50 µl of sample and 50 µl biotinylated Phleum pratense are dispensed by the sample probe into the cuvette. The cuvette reaches the first reagent probe R1, where 100 µl paramagnetic particles with immobilised monoclonal anti-IgE antibody (ALK Laboratories A/S, Hørsholm, Denmark) specific against the IgE Fc domain are dispensed together with 200 µl of streptavidin-acridinium ester label (ALK Laboratories A/S, Hørsholm, Denmark). The cuvette moves down the track to the magnets and wash station. Wash with 750 µl deionized water is performed twice. After completion of the wash cycle the particles are resuspended in 300 µl 0.5 g/l H₂O₂ in 0.1 M HNO₃. The cuvette enters the luminometer chamber and in front of the photomultiplier 300 µl 25 mM NaOH solution is added and the photons of light emitted are measured and quantitated and expressed as relative light units (RLU). The amount of RLU is directly proportional to the amount of the IgE in the sample. The time from sample dispension to first result is 15 min and a new result follows every 20 second. Results were expressed as RLU experiment/RLU background, where RLU background was the chemiluminescent reaction observed in the absence of total IgE.

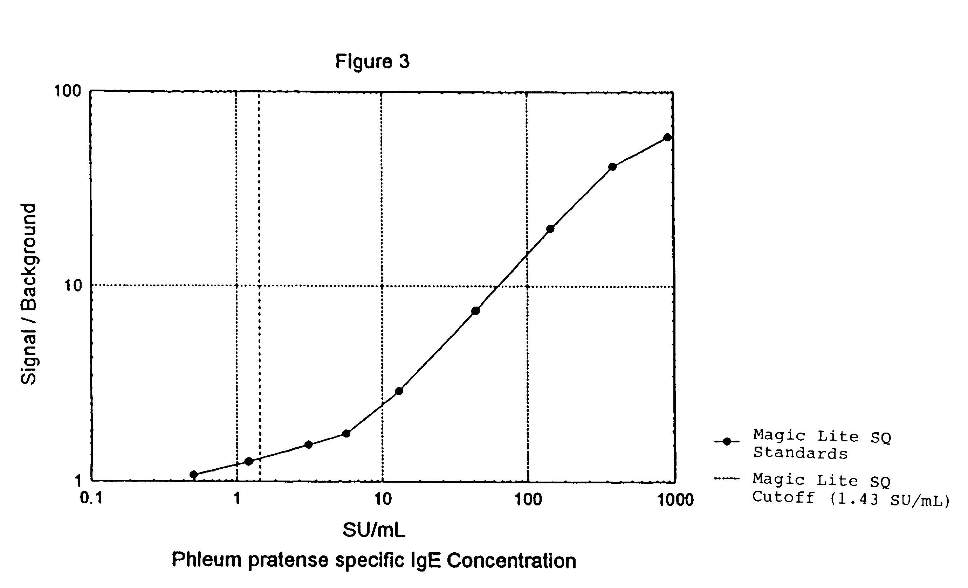

Ten Phleum pratense specific IgE standards, calibrated in Magic Lite SQ Specific IgE Kit (ALK Laboratories A/S, Hørsholm, Denmark) against clinically characterised Phleum pratense allergic patients samples and expressed as SU/ml (Standardised Units), were assayed using the protocol described above and it is shown that between 1.43 and 800 SU/ml of Phleum pratense specific IgE can be measured as in Magic Lite SQ Specific IgE assay, see Figure 3.

### Example 3

### Quantification of specific IgE against WHO Total IgE reference

Quantification of specific IgE antibodies in serum samples were performed with reference to total IgE antibody or specific IgE antibody using the identical assay protocols as described in example one and two, respectively.

Thirtyfive patient samples were assayed for Phleum pratense specific IgE along with 10 Phleum pratense specific IgE standards, calibrated in Magic Lite SQ Specific IgE Kit (ALK Laboratories A/S, Hørsholm, Denmark) against clinically characterised Phleum pratense allergic patient samples and expressed as SU/ml (protocol described in example 2).

Nine standards of IgE WHO 2nd IRP No. 75/502 (National biological standard board) were assayed in the same run against total IgE (protocol described in example 1).

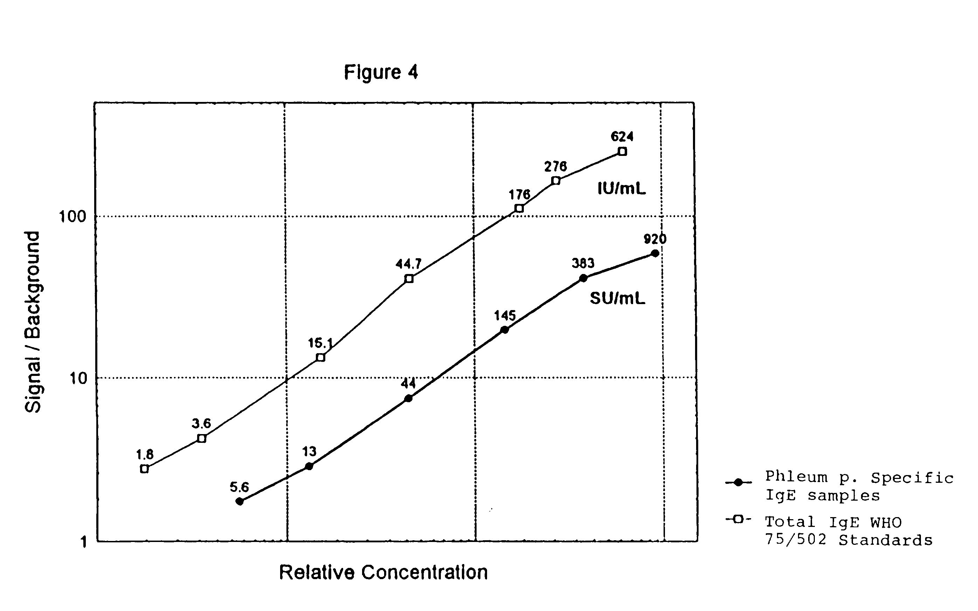

Figure 4 provides a comparison of the two dose response curves of total IgE assay and Phleum pratense specific IgE assay respectively. Parallel line test showed no significant difference in slopes between the two dose response curves, indicating that specific IgE in patient samples could be calibrated against the WHO Total IgE standard and expressed in IU/ml.

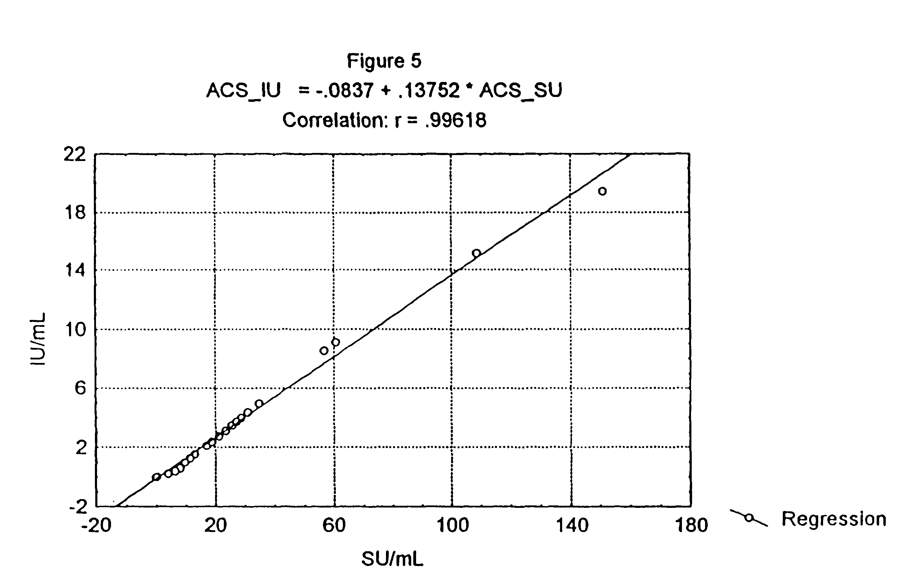

Figure 5 provides a comparison of results from the 35 patient samples calibrated against WHO Total IgE standards (expressed in IU/ml) or Phleum pratense specific IgE standards (expressed in SU/ml) and it shows very good correlation between the two units.

The concentration (dose) of the unknown sample was calculated using a cubic-free spline interpolation after a log vs. log transformation of signal/background and concentration (dose), respectively.

It was calculated from the linear regression line that one SU (Standardised Unit) corresponds to 0.14 International Unit (IU).

In conclusion allergen specific IgE can be measured using the embodiment of the present invention and calibrated directly from the total IgE assay of a WHO IgE calibrated standard curve.

### Example 4

### Total IgE method comparison

Thirtythree patients samples were measured for total serum IgE in the Magic Lite Total IgE kit (ALK Laboratories A/S, Hørsholm, Denmark). The assay was performed according to the manufacturer's instruction. The same samples were measured for total serum IgE on the ACS:180 according to the protocol described in example 1.

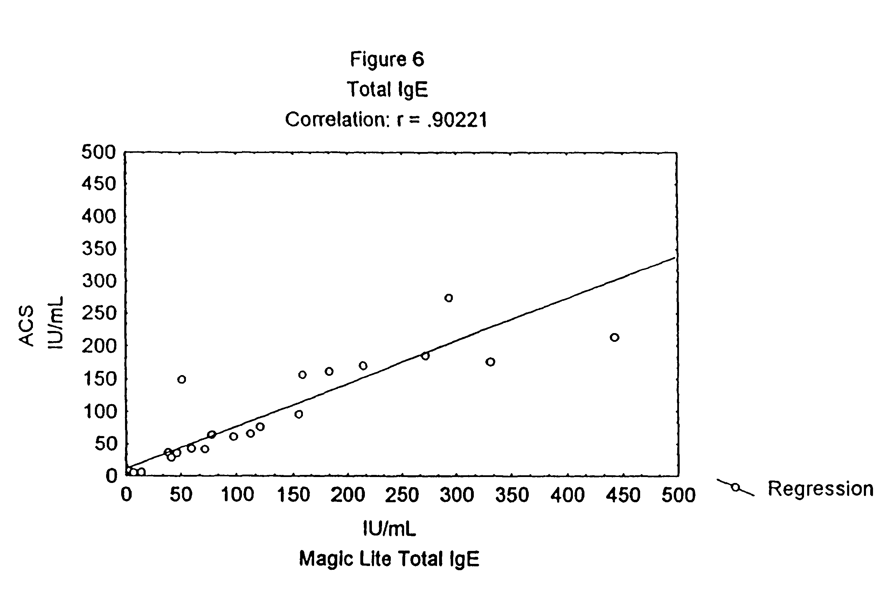

Figure 6 provides a scatterplot for the method comparison for measuring total serum IgE. A correlation with r = 0.90 was found.

### Example 5

### Specific IgE method comparison

Thirtyfive patients samples were measured for Phleum pratense specific IgE in the Magic Lite SQ Specific IgE kit (ALK Laboratories A/S, Hørsholm, Denmark). The assay was performed according to the manufacturer's instruction. The same samples were measured for Phleum pratense specific IgE on the ACS:180 according to the protocol described in example 2.

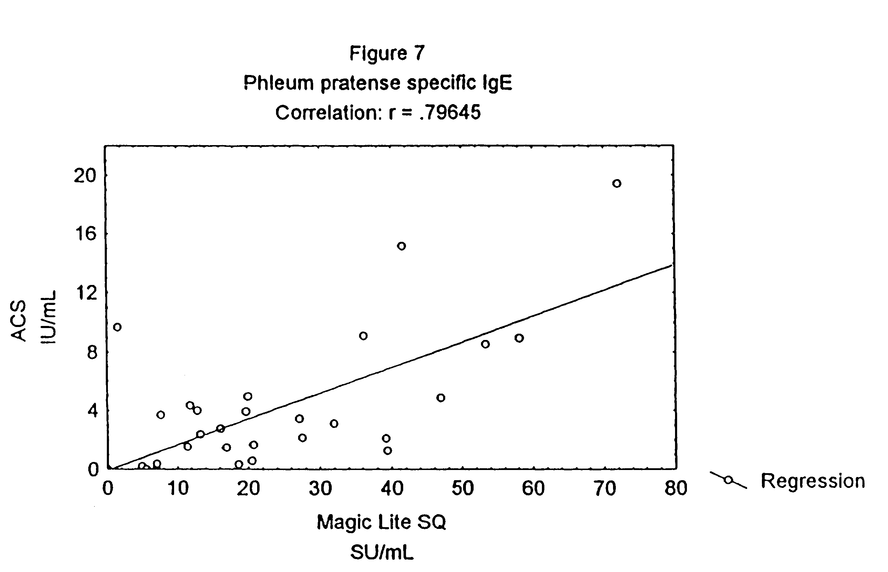

Figure 7 provides a scatterplot for the method comparison for measuring Phleum pratense specific IgE. A correlation with r = 0.80 was found.

### Example 6

### Comparison of assay precision

The within-run imprecision for ACS total IgE using the protocol described in example 1, was compared to that of Magic Lite Total IgE. Patient samples were run in replicates of three in the assays described in example 4. The pooled within-run coefficient of variation (CVpwr) was calculated according to Krouwer and Rabinowitz, Clinical Chemistry, 30, 290 (1984). The following results were obtained:

| | Magic Lite | ACS |
|---|---|---|
| %CVpwr | 4.69 | 2.75 |
| Min %CV | 0.80 | 0.70 |
| Max %CV | 11.70 | 8.40 |

As seen from the results, automation and minimation of operating steps significantly improves the precision of the analysis (by F-test: F=1.71, p=0.049).

### Example 7

### Comparison of total and specific IgE in patient samples:

The samples measured for Phleum pratense specific IgE on the ACS:180 according to the protocol described in example 2 and calibrated against total IgE reference (WHO 75/502) as described in example 3, were also analysed for total IgE as described in example 1. The ratio between measured specific IgE and total IgE was calculated on each sample and expressed as % ratio (spec. IU/total IU* 100).

The following results were obtained:

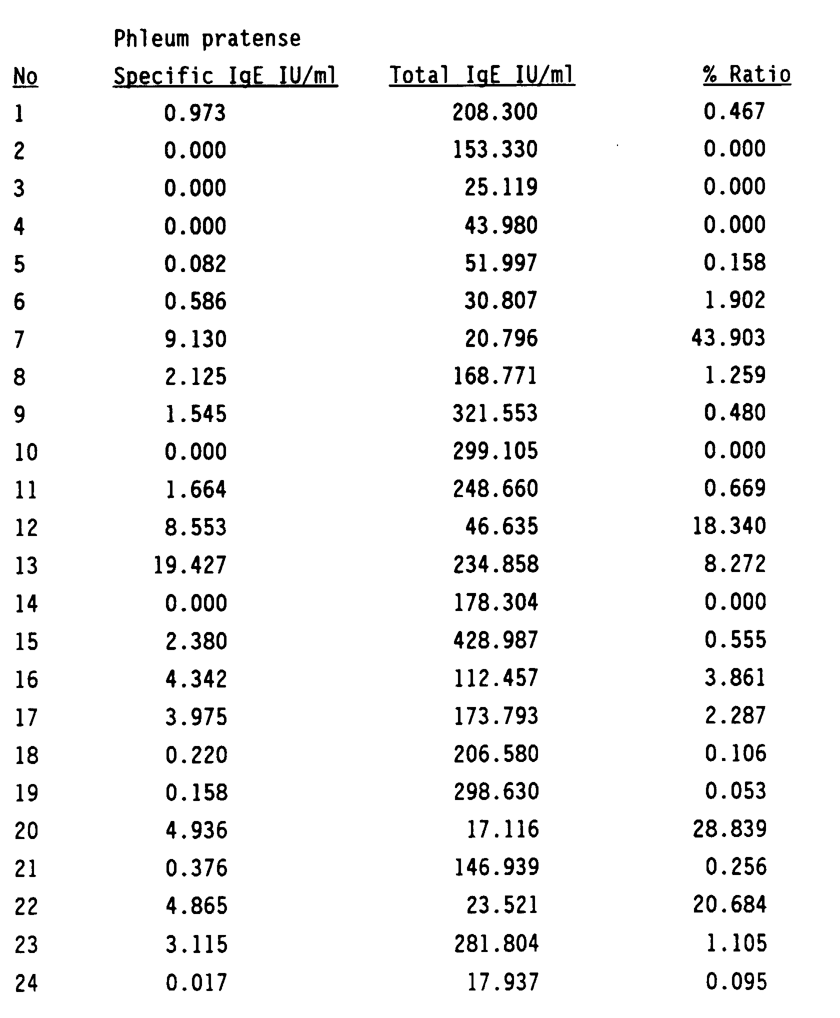

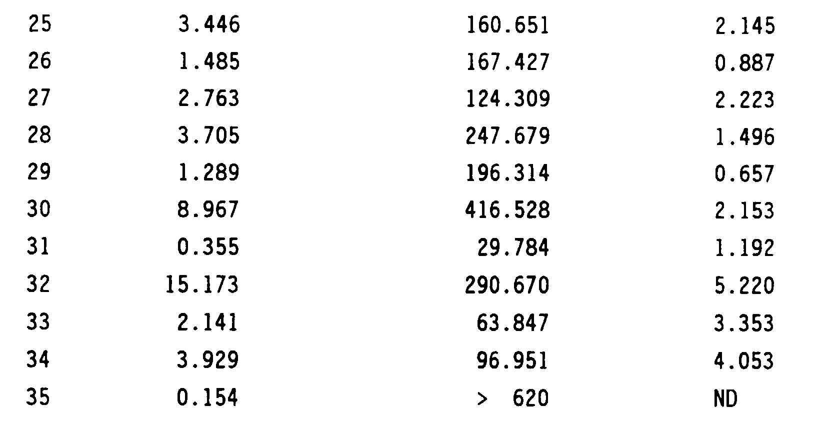

### Example 7 (continued)

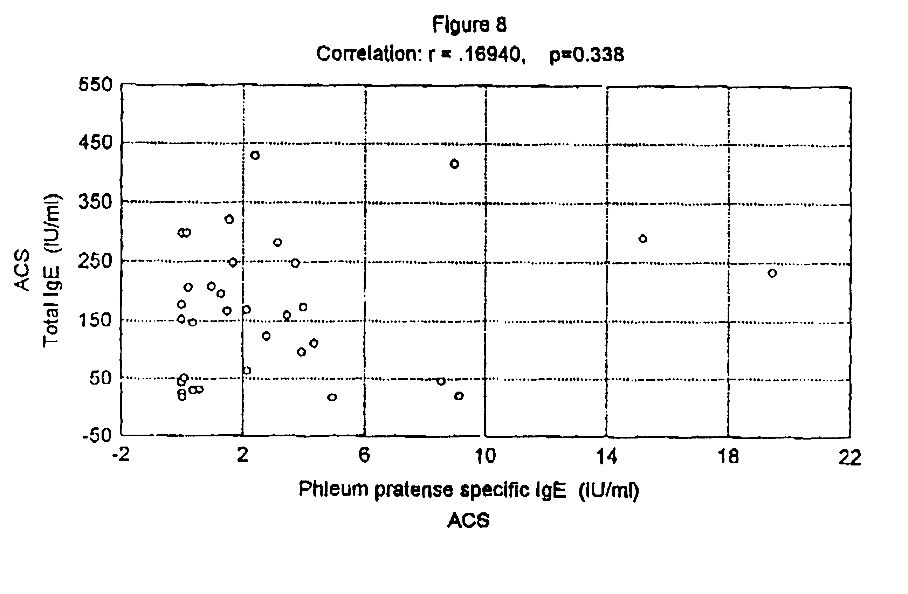

As seen from the results the Phleum pratense specific IgE assay was able to measure as low as 0.05 % of Phleum pratense specific IgE out of total IgE. Low relative amounts of Phleum pratense specific IgE indicates that other allergen specificities are present in the samples. Up to 44 % of total IgE was found in one patient sample to be specific against Phleum pratense. No correlation was found between total IgE concentration and Phleum pratense specific IgE concentration as seen in figure 8.

### Example 8

Determination of serum total IgA antibodies using paramagnetic particles and avidin-acridinium ester label.

The determination of total IgA antibodies was assayed using the following protocol:

25 µl of patient sample or calibrator was pipetted into a 12x75 mm test tube. To each tube 50 µl of biotinylated polyclonal anti-IgA antibody DAKO E484 (supplied by DAKO, Glostrup, Denmark) in 0.05 M phosphate buffer, pH 7.4, containing 0.1 % sodium azide, 0.01 % Tween® 20 and 0.1 % human serum albumin was added and reacted for 15 minutes at ambient temperature. 400 µl slurry of paramagnetic particles with immobilised polyclonal anti-IgA antibody DAKO A 262, (supplied by DAKO, Glostrup, Denmark) was added to each tube and incubated for 5 minutes. After this second incubation 50 µl of streptavidin-acridinium ester label diluted in the same buffer as described above was added to each tube and incubated for a further 5 minutes at ambient temperature. The paramagnetic particles were washed twice with a 0.2 M phosphate buffer, pH 7.4, containing 0.1 % Tween® 20, after separating the magnetic particles from the liquid by a magnetic base separator and vortexing the separated particles with the washing buffer as described above. The contents of the tubes were finally measured in the luminometer, where light emitted at 426 nm, was quantitated and expressed as relative light units (RLUs).

Total IgA standards calibrated against WHO No. 67/86 for human serum IgA DAKO X908 (supplied by DAKO, Glostrup, Denmark) were assayed using the above described protocol.

### Standards:

| Concentration (µg/ml) | RLUs |
|---|---|
| 0 | 370937 |
| 0.02 | 417000 |
| 0.23 | 557563 |
| 2.32 | 1252260 |
| 23.2 | 1872357 |

It should be apparent to one having ordinary skill in the art that many variations are possible without departing from the scope of the invention.

## Claims

1. A method of detecting an antibody in a sample using a chemiluminescent labelling compound, comprising
a) mixing a ligand antigen, antibody, or hapten bound to biotin or a functional derivative thereof; an antibody directed against the antibody to be detected bound to paramagnetic particles; and a chemiluminescent acridinium compound bound to avidin, streptavidin or a functional derivative thereof with the sample to form a solid phase bound complex,
b) magnetically separating the solid phase from the liquid phase,
c) initiating the chemiluminescent reaction, and analysing the separated solid phase for the presence of chemiluminescent complex, wherein the presence of chemiluminescence is an indication of the presence of said antibody in said sample.

2. A method according to claim 1, comprising
i) mixing the ligand antigen, antibody, or hapten bound to biotin or a functional derivative thereof with the sample and the antibody directed against the antibody to be detected bound to paramagnetic particles to form a first solid phase complex,
ii) adding a chemiluminescent acridinium compound covalently bound to avidin, streptavidin or a functional derivative thereof to form a second solid phase complex,
iii) magnetically separating the solid phase from the liquid phase;
iv) initiating the chemiluminescent reaction, and analysing the separated solid phase for the presence of chemiluminescent complex.

3. A method according to claim 1 or 2, wherein the antibody in the sample is a specific immunoglobulin selected from the group consisting of specific IgA, IgD, IgE, IgG, IgM, and isotypes thereof, and the ligand antigen, antibody, or hapten is a specific allergen.

4. A method according to claim 3, wherein the specific immunoglobulin is a specific IgE.

5. A method according to claim 1 or 2, wherein the antibody in the sample is a class of immunoglobulins selected from the group consisting of total IgA, total IgD, total IgE, total IgG, total IgM, and isotypes thereof, and the ligand antigen, antibody or hapten is an antibody directed against said class of immunoglobulins.

6. A method according to claim 5, wherein the class of immunoglobulins is total IgE.

7. A method according to any one of the preceding claims, wherein the antibody directed against the antibody to be detected bound to paramagnetic particles is selected from the group consisting of polyclonal antibodies, monoclonal antibodies including recombinant antibodies, fragmented antibodies; preferably a monoclonal mouse anti-immunoglobulin.

8. A method according to any one of the preceeding claims, wherein the chemiluminescent acridinium compound is N-hydroxysuccinimide dimethylacridinium ester covalently bound to avidin, streptavidin or a functional derivative thereof.

9. A method of measuring the concentration and/or the relative contents of a specific antibody in a sample, wherein the measured light emission of a separated solid phase comprising a captured specific antibody coupled to a chemiluminescent label is compared with the measured light emission obtained in a parallel reference immuno-assay wherein the total contents of the class of antibodies in the sample to which said specific antibody belongs is measured, comprising the steps of
a) mixing a ligand antigen or hapten towards which the specific antibody to be measured is directed bound to biotin or a functional derivative thereof; an antibody directed against the constant portion of the antibody to be measured bound to paramagnetic particles; and a chemiluminescent acridinium compound bound to avidin, streptavidin or a functional derivative thereof with the sample to form a first solid phase complex,
b) magnetically separating said first solid phase from the liquid phase,
c) initiating a chemiluminescent reaction and measuring the light emission of the separated first solid phase,
d) mixing a ligand antibody directed against the class of antibodies to be measured bound to biotin or a functional derivative thereof; an antibody directed against the constant portion of the class of antibodies to be measured bound to paramagnetic particles; and a chemiluminescent acridinium compound bound to avidin, streptavidin or a functional derivative thereof with the sample to form a second solid phase complex,
e) magnetically separating said second solid phase from the liquid phase,
f) initiating the chemiluminescence reaction and measuring the light emission of the separated second solid phase, and
g) comparing the light emission of the separated first solid phase with that of the separated second solid phase.

10. A method according to claim 9, wherein step a) is performed by
(i) mixing the ligand antigen or hepten bound to bioten or a functional derivative thereof with the sample and the antibody bound to paramagnetic particles to form a solid phase complex, and
(ii) adding the chemiluminescent acridinium compound bound to avidin, streptavidin or a functional derivative thereof to form said first solid phase complex,
and wherein step d) is performed by
(i) mixing the ligand antibody bound to biotin or a functional derivative thereof with the sample and the antibody bound to paramagnetic particles to form a solid phase complex and
(ii) adding the chemiluminescent acridinium compound covalently bound to avidin, streptavidin or a functional derivative thereof to form said second solid phase complex.

11. A method according to claim 9 or 10, wherein the specific antibody to be measured in the sample is a specific immunoglobulin selected from the group consisting of IgA, IgD, IgE, IgG, IgM, and isotypes thereof, and the ligand antigen, antibody, or hapten directed against the variable portion of said antibody is an allergen, and the class of antibodies is a class of immunoglobulins selected from the group consisting of total IgA, total IgD, total IgE, total IgG, total IgM, and isotypes thereof, and the ligand antigen, antibody or hapten is an antibody directed against said class of immunoglobulins.

12. A method according to claim 11, wherein the specific immunoglobulin is a specific IgE, and the class of antibodies is total IgE.

13. A method according to claim 9 or 10, wherein the antibody directed against the antibody to be measured bound to paramagnetic particles is selected from the group consisting of polyclonal antibodies, monoclonal antibodies including recombinant antibodies, fragmented antibodies, preferably a monoclonal mouse anti-immunoglobulin.

14. A method according to any one of the preceeding claims, wherein the chemiluminescent acridinium compound is N-hydroxysuccinimide dimethylacridinium ester covalently bound to avidin, streptavidin or a functional derivative thereof.

## Patentansprüche

1. Verfahren zum Nachweis eines Antikörpers in einer Probe unter Verwendung einer chemolumineszenten Markierungsverbindung, welches folgendes umfaßt:
a) Vermischen eines an Biotin oder ein funktionelles Derivat davon gebundenen Antigen-, Antikörper- oder Haptenliganden; eines gegen den zu detektierenden Antikörper gerichteten, an paramagnetische Teilchen gebundenen Antikörpers; und einer an Avidin, Streptavidin oder ein funktionelles Derivat davon gebundenen chemolumineszenten Acridiniumverbindung mit der Probe zur Bildung eines Festphasen-gebundenen Komplexes,
b) magnetisches Trennen der festen Phase von der flüssigen Phase,
c) Einleiten der chemolumineszenten Reaktion und Analysieren der abgetrennten festen Phase hinsichtlich der Gegenwart des chemolumineszenten Komplexes, wobei die Gegenwart der Chemolumineszenz eine Anzeige der Gegenwart des Antikörpers in der Probe ist.

2. Verfahren nach Anspruch 1, welches folgendes umfaßt:
i) Vermischen des an Biotin oder ein funktionelles Derivat davon gebundenen Antigen-, Antikörper- oder Haptenliganden mit der Probe und dem gegen den zu detektierenden Antikörper gerichteten, an paramagnetische Teilchen gebundenen Antikörper zur Bildung eines ersten Festphasen-Komplexes,
ii) Zugeben einer kovalent an Avidin, Streptavidin oder ein funktionelles Derivat davon gebundenen chemolumineszenten Acridiniumverbindung zur Bildung eines zweiten Festphasenkomplexes
iii) magnetisches Trennen der festen Phase von der flüssigen Phase,
iv) Einleiten der chemolumineszenten Reaktion und Analysieren der abgetrennten festen Phase hinsichtlich der Gegenwart des chemolumineszenten Komplexes.

3. Verfahren nach Anspruch 1 oder 2, worin der Antikörper in der Probe ein spezifisches Immunoglobulin ist, gewählt aus der spezifisches IgA, IgD, IgE, IgG, IgM und Isotypen davon umfassenden Gruppe, und der Antigen-, Antikörper- oder Haptenligand ein spezifisches Allergen ist.

4. Verfahren nach Anspruch 3, worin das spezifische Immunoglobulin ein spezifisches IgE ist.

5. Verfahren nach Anspruch 1 oder 2, worin der Antikörper in der Probe eine Immunoglobulinklasse ist, gewählt aus der Gesamt-IgA, Gesamt-IgD, Gesamt-IgE, Gesamt-IgG, Gesamt-IgM und Isotypen davon umfassenden Gruppe, und der Antigen-, Antikörper- oder Haptenligand ein gegen die Immunoglobulinklasse gerichteter Antikörper ist.

6. Verfahren nach Anspruch 5, worin die Immunoglobulinklasse Gesamt-IgE ist.

7. Verfahren nach mindestens einem der vorstehenden Ansprüche, worin der gegen den zu detektierenden Antikörper gerichtete, an paramagnetische Teilchen gebundene Antikörper aus der polyklonale Antikörper, monoklonale Antikörper, einschließlich rekombinanter Antikörper, und fragmentierte Antikörper umfassenden Gruppe gewählt wird, und vorzugsweise ein monoklonales Maus-Anti-Immunoglobulin ist.

8. Verfahren nach mindestens einem der vorstehenden Ansprüche, worin die chemolumineszente Acridiniumverbindung kovalent an Avidin, Streptavidin oder ein funktionelles Derivat davon gebundener N-Hydroxysuccinimiddimethylacridiniumester ist.

9. Verfahren zur Messung der Konzentration und/oder des relativen Gehalts eines spezifischen Antikörpers in einer Probe, worin die gemessene Lichtemission einer abgetrennten festen Phase, welche einen eingefangenen, an eine chemolumineszente Markierung gekoppelten spezifischen Antikörper umfaßt, mit der in einem parallelen Referenz-Immunoassay erhaltenen gemessenen Lichtemission verglichen wird, worin der Gesamtgehalt der Antikörperklasse in der Probe, zu welcher der spezifische Antikörper gehört, gemessen wird, umfassend die folgenden Schritte:
a) Vermischen eines an Biotin oder ein funktionelles Derivat davon gebundenen Antigen- oder Haptenliganden, gegen das der zu messende spezifische Antikörper gerichtet ist; eines gegen den konstanten Abschnitt des zu messenden Antikörpers gerichteten, an paramagnetische Teilchen gebundenen Antikörpers; und einer an Avidin, Streptavidin oder ein funktionelles Derivat davon gebundenen chemolumineszenten Acridiniumverbindung mit der Probe zur Bildung eines ersten Festphasen-Komplexes.
b) magnetisches Trennen der ersten Festphase von der flüssigen Phase,
c) Einleiten der chemolumineszenten Reaktion und Messen der Lichtemission der abgetrennten ersten Festphase,
d) Vermischen eines an Biotin oder ein funktionelles Derivat davon gebundenen, gegen die zu messende Antikörperklasse gerichteten Antikörperliganden; eines gegen den konstanten Abschnitt der zu messenden Antikörperklasse gerichteten, an paramagnetische Teilchen gebundenen Antikörpers; und einer an Avidin, Streptavidin oder ein funktionelles Derivat davon gebundenen chemolumineszenten Acridiniumverbindung mit der Probe zur Bildung eines zweiten Festphasen-Komplexes,
e) magnetisches Trennen der zweiten Festphase von der flüssigen Phase,
f) Einleiten der chemolumineszenten Reaktion und Messen der Lichtemission der abgetrennten zweiten Festphase, und
g) Vergleichen der Lichtemission der abgetrennten ersten Festphase mit derjenigen der abgetrennten zweiten Festphase.

10. Verfahren nach Anspruch 9, worin der Schritt a) durchgeführt wird mittels
(i) Vermischen des an Biotin oder ein funktionelles Derivat davon gebundenen Antigen- oder Haptenliganden mit der Probe und dem an paramagnetische Teilchen gebundenen Antikörper zur Bildung eines Festphasen-Komplexes, und
(ii) Zugeben einer an Avidin, Streptavidin oder ein funktionelles Derivat davon gebundenen chemolumineszenten Acridiniumverbindung zur Bildung des ersten Festphasen-Komplexes,
und worin der Schritt d) durchgeführt wird mittels
(i) Vermischen des an Biotin oder ein funktionelles Derivat davon gebundenen Antikörperliganden mit der Probe und dem an paramagnetische Teilchen gebundenen Antikörper zur Bildung eines Festphasen-Komplexes, und
(ii) Zugeben einer kovalent an Avidin, Streptavidin oder ein funktionelles Derivat davon gebundenen chemolumineszenten Acridiniumverbindung zur Bildung des zweiten Festphasen-Komplexes.

11. Verfahren nach Anspruch 9 oder 10, worin der zu messende spezifische Antikörper in der Probe ein spezifisches Immunoglobulin ist, gewählt aus der IgA, IgD, IgE, IgG, IgM und Isotypen davon umfassenden Gruppe, und der gegen den variablen Abschnitt des Antikörpers gerichtete Antigen-, Antikörper- oder Haptenligand ein Allergen ist, und die Antikörperklasse eine Immunoglobulinklasse ist, gewählt aus der Gesamt-IgA, Gesamt-IgD, Gesamt-IgE, Gesamt-IgG, Gesamt-IgM und Isotypen davon umfassenden Gruppe, und der Antigen-, Antikörper- oder Haptenligand ein gegen die Immunoglobulinklasse gerichteter Antikörper ist.

12. Verfahren nach Anspruch 11, worin das spezifische Immunoglobulin ein spezifisches IgE ist, und die Antikörperklasse Gesamt-IgE ist.

13. Verfahren nach Anspruch 9 oder 10, worin der gegen den zu messenden Antikörper gerichtete, an paramagnetische Teilchen gebundene Antikörper aus der polyklonale Antikörper, monoklonale Antikörper, einschließlich rekombinanter Antikörper, und fragmentierte Antikörper umfassenden Gruppe gewählt wird, und vorzugsweise ein monoklonales Maus-Anti-Immunoglobulin ist.

14. Verfahren nach mindestens einem der vorstehenden Ansprüche, worin die chemolumineszente Acridiniumverbindung kovalent an Avidin, Streptavidin oder ein funktionelles Derivat davon gebundener N-Hydroxysuccinimiddimethylacridiniumester ist.

## Revendications

1. Un procédé pour détecter un anticorps dans un échantillon en utilisant un composé à marquage chimioluminescent, comprenant le fait de
a) mélanger un ligand antigène, anticorps, ou haptène lié à la biotine ou à un de ses dérivés fonctionnels ; un anticorps dirigé contre l'anticorps à détecter lié à des particules paramagnétiques ; et un composé d'acridinium chimioluminescent lié à l'avidine, à la streptavidine ou à un de leurs dérivés fonctionnels avec l'échantillon pour former un complexe lié à une phase solide,
b) séparer magnétiquement la phase solide de la phase liquide,
c) initier la réaction chimioluminescente, et analyser la phase solide séparée pour la présence d'un complexe chimioluminescent, dans lequel la présence de chimioluminescence est une indication de la présence dudit anticorps dans ledit échantillon.

2. Un procédé selon la revendication 1, comprenant le fait de
i) mélanger le ligand antigène, anticorps, ou haptène lié à la biotine ou à un de ses dérivés fonctionnels avec l'échantillon et l'anticorps dirigé contre l'anticorps à détecter lié à des particules paramagnétiques pour former un premier complexe de phase solide,
ii) ajouter un composé d'acridinium chimioluminescent lié de manière covalente à l'avidine, à la streptavidine ou à un de leurs dérivés fonctionnels pour former un deuxième complexe de phase solide,
(iii) séparer magnétiquement la phase solide de la phase liquide,
(iv) initier la réaction chimioluminescente, et analyser la phase solide séparée pour la présence d'un complexe chimioluminescent.

3. Un procédé selon la revendication 1 ou 2, dans lequel l'anticorps dans l'échantillon est une immunoglobuline spécifique choisie dans le groupe constitué des IgA, IgD, IgE, IgG, IgM spécifiques, et de leurs isotypes, et le ligand antigène, anticorps, ou haptène est un allergène spécifique.

4. Un procédé selon la revendication 3, dans lequel l'immunoglobuline spécifique est une IgE spécifique.

5. Un procédé selon la revendication 1 ou 2, dans lequel l'anticorps dans l'échantillon est une classe d'immunoglobulines choisie dans le groupe constitué des IgA totales, des IgD totales, des IgE totales, des IgG totales, des IgM totales, et de leurs isotypes, et le ligand antigène, anticorps ou haptène est un anticorps dirigé contre ladite classe d'immunoglobulines.

6. Un procédé selon la revendication 5, dans lequel la classe d'immunoglobulines est celle des IgE totales.

7. Un procédé selon l'une quelconque des revendications précédentes, dans lequel l'anticorps dirigé contre l'anticorps à détecter lié à des particules paramagnétiques est choisi dans le groupe constitué des anticorps polyclonaux, des anticorps monoclonaux comprenant des anticorps recombinants, des anticorps fragmentés ; de préférence un anti-immunoglobuline monoclonal de souris.

8. Un procédé selon l'une quelconque des revendications précédentes, dans lequel le composé d'acridinium chimioluminescent est l'ester N-hydroxysuccinimide de diméthylacridinium lié de manière covalente à l'avidine, à la streptavidine ou à un de leurs dérivés fonctionnels.

9. Un procédé pour mesurer la concentration et/ou le taux relatif d'un anticorps spécifique dans un échantillon, dans lequel l'émission de lumière mesurée d'une phase solide séparée comprenant un anticorps spécifique capturé couplé à un marqueur chimioluminescent est comparée à l'émission de lumière mesurée obtenue dans un immunodosage parallèle de référence dans lequel on mesure le taux total de la classe d'anticorps dans l'échantillon à laquelle appartient ledit anticorps spécifique, comprenant les étapes consistant à
a) mélanger un ligand antigène ou haptène, contre lequel l'anticorps spécifique à mesurer est dirigé, lié à la biotine ou à un de ses dérivés fonctionnels ; un anticorps dirigé contre la région constante de l'anticorps à mesurer lié à des particules paramagnétiques ; et un composé d'acridinium chimioluminescent lié à l'avidine, à la streptavidine ou à un de leurs dérivés fonctionnels avec l'échantillon pour former un premier complexe de phase solide,
b) séparer magnétiquement ladite première phase solide de la phase liquide,
c) initier une réaction chimioluminescente et mesurer l'émission de lumière de la première phase solide séparée,
d) mélanger un ligand anticorps dirigé contre la classe d'anticorps à mesurer lié à la biotine ou à un de ses dérivés fonctionnels ; un anticorps dirigé contre la région constante de la classe d'anticorps à mesurer lié à des particules paramagnétiques ; et un composé d'acridinium chimioluminescent lié à l'avidine, à la streptavidine ou à un de leurs dérivés fonctionnels avec l'échantillon pour former un deuxième complexe de phase solide,
e) séparer magnétiquement ladite deuxième phase solide de la phase liquide,
f) initier la réaction de chimioluminescence et mesurer l'émission de lumière de la deuxième phase solide séparée, et
g) comparer l'émission de lumière de la première phase solide séparée à celle de la deuxième phase solide séparée.

10. Un procédé selon la revendication 9, dans lequel l'étape a) est effectuée en
(i) mélangeant le ligand antigène ou haptène lié à la biotine ou à un de ses dérivés fonctionnels avec l'échantillon et l'anticorps lié à des particules paramagnétiques pour former un complexe de phase solide, et
(ii) ajoutant le composé d'acridinium chimioluminescent lié à l'avidine, à la streptavidine ou à un de leurs dérivés fonctionnels pour former ledit premier complexe de phase solide,
et dans lequel l'étape d) est effectuée en
(i) mélangeant le ligand anticorps lié à la biotine ou à un de ses dérivés fonctionnels avec l'échantillon et l'anticorps lié à des particules paramagnétiques pour former un complexe de phase solide, et
(ii) ajoutant le composé d'acridinium chimioluminescent lié de manière covalente à l'avidine, à la streptavidine ou à un de leurs dérivés fonctionnels pour former ledit deuxième complexe de phase solide,

11. Un procédé selon la revendication 9 ou 10, dans lequel l'anticorps spécifique à mesurer dans l'échantillon est une immunoglobuline spécifique choisie dans le groupe constitué des IgA, IgD, IgE, IgG, IgM, et de leurs isotypes, et le ligand antigène, anticorps, ou haptène dirigé contre la région variable dudit anticorps est un allergène, et la classe d'anticorps est une classe d'immunoglobulines choisie dans le groupe constitué des IgA totales, des IgD totales, des IgE totales, des IgG totales, des IgM totales, et de leurs isotypes, et le ligand antigène, anticorps ou haptène est un anticorps dirigé contre ladite classe d'immunoglobulines.

12. Un procédé selon la revendication 11, dans lequel l'immunoglobuline spécifique est une IgE spécifique, et la classe d'anticorps est celle des IgE totales.

13. Un procédé selon la revendication 9 ou 10, dans lequel l'anticorps dirigé contre l'anticorps à mesurer lié à des particules paramagnétiques est choisi dans le groupe constitué des anticorps polyclonaux, des anticorps monoclonaux comprenant des anticorps recombinants, des anticorps fragmentés, de préférence un anti-immunoglobuline monoclonal de souris.

14. Un procédé selon l'une quelconque des revendications précédentes, dans lequel le composé d'acridinium chimioluminescent est l'ester N-hydroxysuccinimide de diméthylacridinium lié de manière covalente à l'avidine, à la streptavidine ou à un de leurs dérivés fonctionnels.
